# EUROPEAN PATENT APPLICATION

(11) **EP 4 501 898 A1**
(43) Date of publication of application: **05.02.2025**
(21) Application number: 23779518.2
(22) Date of filing: 13.03.2023
(51) Int. Cl.: C07C 51/15, C07C 51/43, C07C 65/105

(54) **METHOD FOR PRODUCING 4,4'-DIHYDROXYBIPHENYL-3,3'-DICARBOXYLIC ACID**

(30) Priority: 30.03.2022 JP 2022057066
(71) Applicant: Ueno Fine Chemicals Industry, Ltd., Tokyo 102-0093 (JP)
(72) Inventor: SHIBA, Ikkyu, Osaka-shi, Osaka 541-8543 (JP); MOTOOKA, Ryota, Osaka-shi, Osaka 541-8543 (JP); SHIMIZU, Shogo, Osaka-shi, Osaka 541-8543 (JP); TANAKA, Kumiko, Osaka-shi, Osaka 541-8543 (JP)
(74) Representative: Kador & Partner Part mbB
(86) International application number: PCT/JP2023/009686
(87) International publication number: WO 2023/189498

(57) **Abstract**

An object of the present invention is to provide a method for producing 4,4'-dihydroxybiphenyl-3,3'-dicarboxylic acid in a high yield. Disclosed is a method for producing 4,4'-dihydroxybiphenyl-3,3'-dicarboxylic acid represented by formula (2), 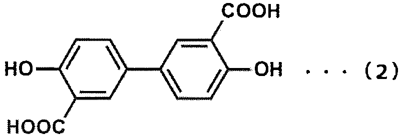 the method comprising a step of reacting a 4,4'-dihydroxybiphenyl dialkali metal salt represented by formula (1), 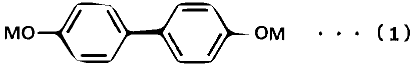 wherein M represents sodium, lithium, rubidium, or cesium, with carbon dioxide in 2-ethylhexanol.

## Description

### TECHNICAL FIELD

The present invention relates to a method for producing 4,4'-dihydroxybiphenyl-3,3'-dicarboxylic acid.

### BACKGROUND ART

Carboxylated biphenol compounds are compounds useful as raw materials for producing aromatic polyesters superior in thermal characteristics, mechanical characteristics, and the like. In recent years, applications as organic ligands for metal-organic frameworks (MOFs) used for gas storage, solid catalysts, and the like are expected. MOFs are one of the porous materials in which a metal and an organic ligand are combined, and Patent Documents 1 and 2 propose to use 4,4'-dihydroxybiphenyl-3,3'-dicarboxylic acid (BPDC) as an organic ligand.

MOFs containing BPDC are attracting attention because of their high carbon dioxide adsorptivity, and as the demand for MOFs increases, the demand for BPDC also increases.

As a method for producing BPDC, a so-called Kolbe-Schmitt reaction is known, and Patent Document 3 describes a method for producing BPDC by reacting 4,4'-dihydroxybiphenyl disodium salt with carbon dioxide in benzyl alcohol.

### PRIOR ART DOCUMENTS

### PATENT DOCUMENTS

Patent Document 1: JP-A-2020-527455
Patent Document 2: JP-A-2020-531248
Patent Document 3: JP-A-H11-140015

### SUMMARY OF THE INVENTION

### PROBLEMS TO BE SOLVED BY THE INVENTION

However, the method described in Patent Document 3 affords a low yield and is not suitable for production on an industrial scale. Therefore, a method for producing BPDC in a high yield has been required.

An object of the present invention is to provide a production method which can yield BPDC in a high yield.

### SOLUTIONS TO THE PROBLEMS

As a result of intensive studies in view of the above problems, the present inventors have found that BPDC is obtained at a high generation rate by reacting a 4,4'-dihydroxybiphenyl dialkali metal salt with carbon dioxide in 2-ethylhexanol, and have accomplished the present invention.

That is, the present invention includes the following preferred embodiments.
[1] A method for producing 4,4'-dihydroxybiphenyl-3,3'-dicarboxylic acid represented by formula (2), 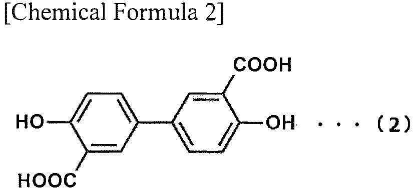 the method comprising a step of reacting a 4,4'-dihydroxybiphenyl dialkali metal salt represented by formula (1), 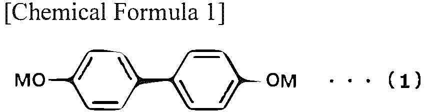 wherein M represents sodium, lithium, rubidium, or cesium, with carbon dioxide in 2-ethylhexanol.
[2] The method according to [1], wherein M is sodium.
[3] The method according to [1] or [2], wherein the reaction of the 4,4'-dihydroxybiphenyl dialkali metal salt with carbon dioxide is performed under the condition of a carbon dioxide pressure of 0.1 to 10 MPa.
[4] The method according to any one of [1] to [3], wherein the reaction step is performed under a condition of a temperature of 100 to 300°C.
[5] The method according to any one of [1] to [4], wherein the reaction step is performed in the presence of an organic acid and/or an organic acid salt.
[6] The method according to [5], wherein the organic acid and/or the organic acid salt is one or more selected from the group consisting of formic acid, acetic acid, propionic acid, oxalic acid, malonic acid, succinic acid, glutamic acid, adipic acid, maleic acid, fumaric acid, phthalic acid, tartaric acid, citric acid, and alkali metal salts thereof.
[7] The method according to [6], wherein the organic acid salt is sodium acetate.
[8] The method according to any one of [1] to [7], comprising a step of adding water to a reaction liquid obtained in the reaction step, then separating the obtained mixture into an organic layer and an aqueous layer, and conducting acid precipitation of the obtained aqueous layer to obtain a crude composition containing 4,4'-dihydroxybiphenyl-3,3'-dicarboxylic acid represented by the formula (2).
[9] The method according to [8], comprising a step of further purifying the crude composition.
[10] The method according to [9], wherein the purification step includes one or more steps selected from the group consisting of suspension washing, recrystallization, and reprecipitation.
[11] The method according to [9] or [10], wherein the purification step includes a suspension washing step.
[12] The method according to any one of [9] to [11], wherein the solvent to be used in the purification step is one or more selected from the group consisting of water, methanol, ethanol, n-propyl alcohol, isopropyl alcohol, 2-ethylhexanol, ethylene glycol, glycerin, acetic acid, N-methyl-2-pyrrolidone, N,N-dimethylformamide, acetonitrile, acetone, xylene, toluene, methyl ethyl ketone, methyl isobutyl ketone, hexane, heptane, cyclohexanone, tetrahydrofuran, 4-methyltetrahydropyran, chloroform, dioxane, ethyl acetate, propyl acetate, and butyl acetate.
[13] 4,4'-Dihydroxybiphenyl-3,3'-dicarboxylic acid represented by the formula (2), wherein the content of potassium is less than 100 ppm.

### EFFECTS OF THE INVENTION

According to the present invention, BPDC can be obtained at a high generation rate.

### DETAILED DESCRIPTION

In the method for producing BPDC represented by the formula (2) of the present invention, a so-called Kolbe-Schmitt reaction in which the 4,4'-dihydroxybiphenyl dialkali metal salt represented by the formula (1) is reacted with carbon dioxide is used. In the present invention, the reaction is usually carried out with stirring. 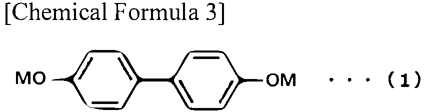 wherein M represents sodium, lithium, rubidium or cesium.

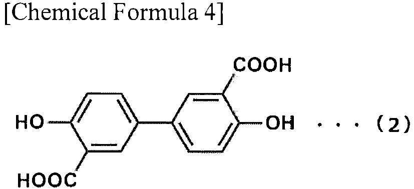

The reactor to be used in the present invention may be any reactor used in an ordinary Kolbe-Schmitt reaction, and for example, an autoclave equipped with a stirrer and capable of coping with a high-pressure reaction can be suitably used. Furthermore, the reactor is more preferably one having a temperature control function and having an introduction tube for carbon dioxide gas or inert gas, a thermometer support tube, a pressure gauge, an exhaust pipe, and the like.

Examples of the 4,4'-dihydroxybiphenyl dialkali metal salt represented by the formula (1) to be used in the present invention include 4,4'-dihydroxybiphenyl disodium salt, 4,4'-dihydroxybiphenyl dilithium salt, 4,4'-dihydroxybiphenyl dirubidium salt, and 4,4'-dihydroxybiphenyl dicesium salt. From the viewpoint of easy availability, cost, and reactivity, 4,4'-dihydroxybiphenyl disodium salt is preferable.

The 4,4'-dihydroxybiphenyl dialkali metal salt can be obtained by converting 4,4'-dihydroxybiphenyl into a dialkali metal salt thereof using an alkali metal hydroxide or an alkali metal alkoxide such as alkali metal tert-butoxide, alkali metal methoxide, alkali metal ethoxide, or alkali metal iso-propoxide. In particular, in consideration of economic efficiency, it is preferable to use 4,4'-dihydroxybiphenyl and an alkali metal hydroxide to form a dialkali metal salt.

More specifically, the 4,4'-dihydroxybiphenyl disodium salt can be obtained by converting 4,4'-dihydroxybiphenyl into a disodium salt thereof using sodium hydroxide or a sodium alkoxide such as sodium tert-butoxide, sodium methoxide, sodium ethoxide, or sodium iso-propoxide. In particular, in consideration of economic efficiency, it is preferable to form a disodium salt using 4,4'-dihydroxybiphenyl and sodium hydroxide.

The 4,4'-dihydroxybiphenyl dialkali metal salt to be subjected to the reaction is preferably sufficiently dehydrated, and when the dehydration is incomplete, the reaction yield may decrease. The dehydration is performed, for example, by heating in a vacuum state using such a device as an evaporator. The 4,4'-dihydroxybiphenyl dialkali metal salt subjected to the reaction preferably has a water content of 3 mass% or less, preferably 2 mass% or less, and more preferably 1 mass% or less.

In the present invention, the reaction of 4,4'-dihydroxybiphenyl dialkali metal salt with carbon dioxide may be carried out in the presence of an organic acid and/or an organic acid salt.

Examples of the organic acid and/or the organic acid salt include one or more selected from the group consisting of acetic acid, formic acid, propionic acid, oxalic acid, malonic acid, succinic acid, glutamic acid, adipic acid, maleic acid, fumaric acid, phthalic acid, tartaric acid, citric acid, and alkali metal salts thereof. In particular, sodium formate and sodium acetate are preferable in consideration of availability, price, and reactivity, and sodium acetate is most preferable in consideration of safety.

When an organic acid and/or an organic acid salt is added, it is preferable that the organic acid and/or the organic acid salt be present in an amount of usually 0.1 to 10 mol, preferably 0.3 to 5 mol, more preferably 0.5 to 4.5 mol, and still more preferably 1 to 3 mol, per 1 mol of the 4,4'-dihydroxybiphenyl dialkali metal salt.

In the method of the present invention, 2-ethylhexanol (EHA) is used as a solvent. By using 2-ethylhexanol as a solvent, the generation rate of BPDC is greatly improved.

The use amount of the 2-ethylhexanol to be used in the present invention is preferably 100 to 3000 parts by mass based on 100 parts by mass of the 4,4'-dihydroxybiphenyl dialkali metal salt represented by the formula (1) (1 to 30 mass times based on the 4,4'-dihydroxybiphenyl dialkali metal salt), more preferably 150 to 2000 parts by mass, still more preferably 200 to 1500 parts by mass, and particularly preferably 300 to 1000 parts by mass. When the use amount of the 2-ethylhexanol is within the above range, the generation rate of BPDC can be efficiently improved.

The reaction of the 4,4'-dihydroxybiphenyl dialkali metal salt with carbon dioxide is carried out usually at 100 to 300°C, and can be carried out preferably at a temperature of 150 to 280°C, and more preferably at a temperature of 170 to 250°C. At a temperature lower than 100°C, the reaction tends to hardly proceed, and at a temperature higher than 300°C, the reaction reaches a plateau, so that energy is lost, and side reactions such as decomposition may occur.

The reaction of the 4,4'-dihydroxybiphenyl dialkali metal salt with carbon dioxide is carried out usually under a pressure of 10 MPa or less, preferably 0.1 to 5 MPa, more preferably 0.2 to 1 MPa, and preferably under a pressure with carbon dioxide. If the pressure during the reaction exceeds 10 MPa, it may be not industrially advantageous, for example, a device that withstands high pressure is required.

The reaction time can be appropriately selected usually between 30 minutes and 24 hours, preferably between 1 hour and 20 hours, more preferably between 2 hours and 16 hours, and particularly preferably between 3 to 12 hours.

The dialkali metal salt of BPDC obtained by such a reaction can afford the targeted BPDC by being converted into an acid by means known to those skilled in the art, such as acid precipitation.

Before the acid precipitation, washing with an organic solvent, a filtration treatment for removing insoluble foreign matters, and an adsorbent treatment with activated carbon or the like for removing a coloring substance, a metal, or the like may be performed in a state where the dialkali metal salt of BPDC is dissolved in a water-soluble solvent, as necessary.

By the step of reacting the 4,4'-dihydroxybiphenyl dialkali metal salt represented by the formula (1) with carbon dioxide in 2-ethylhexanol, a reaction liquid containing the BPDC dialkali metal salt is obtained.

Water is added to the reaction liquid containing the BPDC dialkali metal salt (the liquid after the addition is sometimes referred to as reaction liquid), and the aqueous layer obtained by liquid separation is subjected to acid precipitation with an acid to afford a crude composition containing BPDC.

Before the acid precipitation, a water-soluble medium may be further added separately to the aqueous layer. Examples of the water-soluble medium that can be added include one or more selected from the group consisting of methanol, ethanol, n-propyl alcohol, isopropyl alcohol, ethylene glycol, glycerin, N-methyl-2-pyrrolidone, N,N-dimethylformamide, acetonitrile, acetone, tetrahydrofuran, and dioxane, and it is preferable to add methanol from the viewpoint of making it possible to improve the filterability at the time of solid-liquid separation after the acid precipitation. In addition, for example, when methanol is added, the aqueous layer to be subjected to acid precipitation is preferably prepared to be an aqueous solution containing 20 to 80 mass% of methanol, more preferably an aqueous solution containing 35 to 65 mass% of methanol, and still more preferably an aqueous solution containing 45 to 55 mass% of methanol.

The amount of the medium of the solution to be subjected to the acid precipitation is preferably 1 to 20 mass times, more preferably 2 to 15 mass times, and still more preferably 3 to 10 mass times the mass of BPDC.

The temperature during the acid precipitation is preferably 20 to 95°C, more preferably 30 to 90°C, and still more preferably 40 to 85°C. When the temperature during the acid precipitation is within the above range, side reactions are less prone to occur, and the filterability at the time of solid-liquid separation after the acid precipitation is improved.

In the present invention, the acid to be used for the acid precipitation is not particularly limited, but a mineral acid is suitably used. Examples of the mineral acid include binary acids such as hydrochloric acid and hydrofluoric acid, and oxoacids such as sulfuric acid, nitric acid, phosphoric acid, and perchloric acid. Organic acids such as acetic acid and propionic acid can also be used.

The acid to be used for the acid precipitation is preferably in the form of an aqueous solution from the viewpoint of easily controlling the reaction temperature and the like, and for example, a 62 mass% aqueous sulfuric acid solution is suitably used.

The crude composition containing BPDC means a composition containing impurities such as a reaction raw material, a catalyst, and a reaction by-product in addition to BPDC as a target product. The content of impurities varies depending on the reaction method, but is usually 1 to 40 mass% in the crude composition, and is 3 to 30 mass% in another case. In the present description, the crude composition containing BPDC is also simply referred to as "crude BPDC".

The obtained crude composition containing BPDC may be further subjected to a purification step to have a higher purity.

The purification step preferably includes one or more steps selected from the group consisting of suspension washing, recrystallization, and reprecipitation, and more preferably includes a suspension washing step.

Examples of the solvent to be used in the purification step include one or more selected from the group consisting of water, methanol, ethanol, n-propyl alcohol, isopropyl alcohol, 2-ethylhexanol, ethylene glycol, glycerin, acetic acid, N-methyl-2-pyrrolidone, N,N-dimethylformamide, acetonitrile, acetone, xylene, toluene, methyl ethyl ketone, methyl isobutyl ketone, hexane, heptane, cyclohexanone, tetrahydrofuran, 4-methyltetrahydropyran, chloroform, dioxane, ethyl acetate, propyl acetate, and butyl acetate.

The suspension washing step is performed by adding a suspension washing solvent to the crude composition containing BPDC and stirring the mixture in a suspended state.

The amount of the solvent in the suspension washing varies depending on the type of the solvent, but the amount of the solvent is preferably 1 to 50 mass times, more preferably 3 to 40 mass times, and most preferably 5 to 30 mass times the amount of the crude composition containing BPDC.

The temperature at the time of the suspension washing is not particularly limited because it varies depending on the type and mixing ratio of the solvent to be used, but is preferably 15°C to 200°C, more preferably 20°C to 150°C, and still more preferably 30°C to 100°C.

The recrystallization step is performed by dissolving the crude composition containing BPDC in a recrystallization solvent and then crystallizing the solution.

The amount of the solvent in the recrystallization step varies depending on the type of the solvent, but the amount of the solvent is preferably 1 to 50 mass times, more preferably 3 to 40 mass times, and most preferably 5 to 30 mass times the amount of the crude composition containing BPDC.

In the recrystallization, the temperature at which the crude composition containing BPDC is dissolved in the solvent is not particularly limited because it varies depending on the type and mixing ratio of the solvent to be used, but is preferably 20 to 200°C, more preferably 40 to 150°C, and still more preferably 60 to 130°C.

Crystallization in the recrystallization step is performed with stirring at a temperature of preferably 0 to 80°C, more preferably 5 to 60°C, and still more preferably 10 to 40°C. Incidentally, it is preferable to remove insoluble matters by filtration before the crystallization.

The reprecipitation step is performed by adding a poor solvent to a solution obtained by dissolving the crude composition containing BPDC in a good solvent to precipitate a solid of BPDC.

The good solvent and the poor solvent to be used in the reprecipitation step each can be appropriately selected from the solvents to be used in the purification step.

After the purification step, solid-liquid separation is performed by a conventional means such as filtration to recover BPDC as the target product. In the solid-liquid separation, it is preferable to wash crystals by appropriately pouring an organic solvent or water. The solvent to be used for washing in the solid-liquid separation is preferably 1 to 50 mass times, more preferably 3 to 40 mass times, and most preferably 5 to 30 mass times the crude BPDC.

The crystals recovered by the solid-liquid separation are forced air-dried under normal pressure or dried under reduced pressure, and the solvent is distilled off, whereby highly pure BPDC can be obtained.

The highly pure BPDC obtained by the method of the present invention has a low alkali metal content, which is less than 1,000 ppm, preferably less than 500 ppm, more preferably less than 300 ppm, and still more preferably less than 250 ppm. Such an alkali metal may exist in the form of a hydrochloride, a sulfate, a nitrate, a phosphate, and the like. Examples of the alkali metal include sodium and/or potassium.

Thus, the present invention provides BPDC having an alkali metal content of less than 1,000 ppm, preferably less than 500 ppm, more preferably less than 300 ppm, and still more preferably less than 250 ppm. Examples of the alkali metal include sodium and/or potassium. In one embodiment of the present invention, the content of the alkali metal is usually 5 ppm or more.

The highly pure BPDC obtained by the method of the present invention provides BPDC which has a low content of potassium especially among the alkali metals, wherein the content of potassium is less than 100 ppm, preferably less than 75ppm, and more preferably less than 50 ppm. In one embodiment of the present invention, the content of potassium is usually 1 ppm or more.

### Examples

In the following, the present invention is described in detail by way of Examples, but the present invention is not limited to them.

Each compound was analyzed by the following method.

### <Ultra high performance liquid chromatograph (UPLC)>

Instrument: Waters UPLC H-Class System
Model of column: ACQUITY UPLC HSS C18 1.8 µm 2.1 × 50 mm
Liquid flow rate: 0.5 ml/min
Solvent ratio: H₂O (pH2.3)/methanol = 55/45 (2.5 min) → 0.5 min → 38/62 (1.5 min) → 0.5 min → 20/80 (2 min) → 0.5 min → 5/95 (2.5 min), gradient analysis
Wavelength: 254 nm
Column temperature: 40°C

### [Example 1]

A 120 mL autoclave was charged with 13.3 g (0.06 mol) of 4,4'-dihydroxybiphenyl disodium salt (BP-Na2) and 66.6 g of 2-ethylhexanol (EHA), sealed, and then heated to 200°C with stirring. After reaching 200°C, a reaction was carried out for 7 hours under the condition of carbon dioxide pressure of 0.6 MPa. After completion of the reaction, the mixture was cooled to 80°C, 254.2 g of water was added, and the resulting mixture was stirred and allowed to stand, affording a reaction liquid.

The reaction liquid obtained was subjected to quantitative analysis using UPLC for the conversions of BPDC and BPMC (4,4'-dihydroxybiphenyl-3-carboxylic acid) as an intermediate, and the residual rate of BP (4,4'-dihydroxybiphenyl). The results are shown in Table 1.

### [Example 2]

A reaction liquid was obtained in the same manner as in Example 1 except that the carbon dioxide pressure was changed to 0.9 MPa. The reaction liquid obtained was subjected to quantitative analysis for the conversions to BPDC and BPMC and the residual rate of BP by UPLC. The results are shown in Table 1.

### [Example 3]

A reaction liquid was obtained in the same manner as in Example 1 except that 9.5 g (0.1 mol, 2.0 eq/BP-Na2) of sodium acetate was added. The reaction liquid obtained was subjected to quantitative analysis for the conversions to BPDC and BPMC and the residual rate of BP by UPLC. The results are shown in Table 1.

### [Comparative Example 1]

A reaction liquid was obtained in the same manner as in Example 1 except that EHA was changed to benzyl alcohol (BnOH). The reaction liquid obtained was subjected to quantitative analysis for the conversions to BPDC and BPMC and the residual rate of BP by UPLC. The results are shown in Table 1.

### [Comparative Example 2]

A reaction liquid was obtained in the same manner as in Example 2 except that EHA was changed to benzyl alcohol (BnOH). The reaction liquid obtained was subjected to quantitative analysis for the conversions to BPDC and BPMC and the residual rate of BP by UPLC. The results are shown in Table 1.

### [Comparative Example 3]

A reaction liquid was obtained in the same manner as in Example 1 except that EHA was changed to N, N-dimethylformamide (DMF). The reaction liquid obtained was subjected to quantitative analysis for the conversions to BPDC and BPMC and the residual rate of BP by UPLC. The results are shown in Table 1.

### [Comparative Example 4]

A reaction liquid was obtained in the same manner as in Example 2 except that EHA was changed to N, N-dimethylformamide (DMF). The reaction liquid obtained was subjected to quantitative analysis for the conversions to BPDC and BPMC and the residual rate of BP by UPLC. The results are shown in Table 1.

### [Comparative Example 5]

A reaction liquid was obtained in the same manner as in Example 1 except that EHA was changed to N-methyl-2-pyrrolidone (NMP). The reaction liquid obtained was subjected to quantitative analysis for the conversions to BPDC and BPMC and the residual rate of BP by UPLC. The results are shown in Table 1.

### [Comparative Example 6]

A reaction liquid was obtained in the same manner as in Example 2 except that EHA was changed to N-methyl-2-pyrrolidone (NMP). The reaction liquid obtained was subjected to quantitative analysis for the conversions to BPDC and BPMC and the residual rate of BP by UPLC. The results are shown in Table 1.

### [Example 4]

### <Acquisition of crude BPDC>

The reaction liquid obtained in Example 1 was subjected to liquid-liquid separation, and only the aqueous layer was taken out. The aqueous layer taken out was transferred to a 500 mL four-neck flask with a bottom outlet equipped with a stirrer and a temperature controller, and the temperature was raised to 90°C with stirring. After the raising of the temperature, 72 mass% sulfuric acid was added until the pH reached 8.0, and then washing was performed twice with 65.9 g of 2-ethylhexanol (EHA). To the aqueous layer obtained was added 72 mass% sulfuric acid until the pH reached 2.0, thereby precipitating crude BPDC. The crude BPDC precipitated was collected by filtration and then washed with 115.5 g of water. The crude BPDC obtained was forced air-dried at 80°C, affording 13.9 g of crude BPDC (yield: 94.1 mol%). The crude BPDC obtained was subjected to quantitative analysis by UPLC and crystal inductively coupled plasma (ICP) emission spectrometry. The composition is shown in Table 2.

### [Example 5]

### <Suspension washing step>

The crude BPDC obtained in Example 4 was transferred to a 500 mL four-neck flask equipped with a stirrer and a temperature controller, 351.3 g of a 50% aqueous MeOH solution was added thereto, and suspension washing was performed at 70°C for 1 hour under a nitrogen stream. The suspension was subjected to solid-liquid separation, washed with 130.0 g of water, and the collected crystals were forced air-dried at 80°C, affording 9.7 g of BPDC (yield: 90.5 mol%). The composition of the BPDC obtained is shown in Table 2.

**[Table 1]**

| | Solvent | Additives | | CO₂ pressure | Conversion [mol%] | | Residual ratio [mol%] |
|---|---|---|---|---|---|---|---|
| | | Type | Equivalent [/BP salt] | [MPa] | BPDC | BPMC | BP |
| Example 1 | EHA | - | - | 0.6 | 76.8 | 15.8 | 9.7 |
| Example 2 | EHA | - | - | 0.9 | 77.9 | 10.4 | 11.7 |
| Example 3 | EHA | Na acetate | 2.0 | 0.6 | 79.7 | 4.4 | 24.3 |
| Comparative Example 1 | BnOH | - | - | 0.6 | 58.1 | 13.3 | 18.4 |
| Comparative Example 2 | BnOH | - | - | 0.9 | 65.6 | 7.2 | 15.7 |
| Comparative Example 3 | DMF | - | - | 0.6 | 3.2 | 22.7 | 74.1 |
| Comparative Example 4 | DMF | - | - | 0.9 | 16.1 | 46.5 | 37.4 |
| Comparative Example 5 | NMP | - | - | 0.6 | 10.2 | 43.4 | 46.5 |
| Comparative Example 6 | NMP | - | - | 0.9 | 4.7 | 36.1 | 59.2 |

| | | | | | | | |
|---|---|---|---|---|---|---|---|
| Equivalent: molar amount of additive with respect to molar amount of 4,4'-dihydroxybiphenyl dialkali metal salt | | | | | | | |

**[Table 2]**

| | Solvent | Composition of crystals | | | | |
|---|---|---|---|---|---|---|
| | | BPDC [mass%] | BPMC [mass%] | BP [mass%] | Na [ppm] | K [ppm] |
| Crude BPDC | - | 72.2 | 27.8 | Less than 0.1 | 4156.9 | 1012.7 |
| Example 5 | Aqueous solution containing 50 mass% of methanol | 97.2 | 2.8 | Less than 0.1 | 116.9 | 30.0 |

Table 1 shows that in Examples 1 to 3 of the present invention performed using 2-ethylhexanol solvent were attained greatly improved generation rates of BPDC as compared with Comparative Examples performed using solvents other than 2-ethylhexanol. Example 3 shows that the intermediate is significantly suppressed by adding sodium acetate. In addition, as shown in Table 2, it is found that impurities can be significantly removed by further performing a purification step.

## Claims

1. A method for producing 4,4'-dihydroxybiphenyl-3,3'-dicarboxylic acid represented by formula (2), 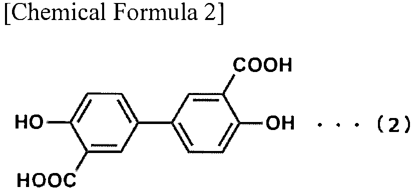 the method comprising a step of reacting a 4,4'-dihydroxybiphenyl dialkali metal salt represented by formula (1), 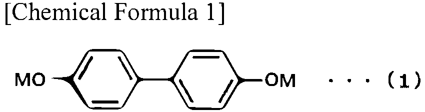 wherein M represents sodium, lithium, rubidium, or cesium, with carbon dioxide in 2-ethylhexanol.

2. The method according to claim 1, wherein M is sodium.

3. The method according to claim 1, wherein a reaction of the 4,4'-dihydroxybiphenyl dialkali metal salt with carbon dioxide is performed under a condition of a carbon dioxide pressure of 0.1 to 10 MPa.

4. The method according to claim 1, wherein a reaction step is performed under a condition of a temperature of 100 to 300°C.

5. The method according to claim 1, wherein the reaction step is performed in a presence of an organic acid and/or an organic acid salt.

6. The method according to claim 5, wherein the organic acid and/or the organic acid salt is one or more selected from a group consisting of formic acid, acetic acid, propionic acid, oxalic acid, malonic acid, succinic acid, glutamic acid, adipic acid, maleic acid, fumaric acid, phthalic acid, tartaric acid, citric acid, and alkali metal salts thereof.

7. The method according to claim 6, wherein the organic acid salt is sodium acetate.

8. The method according to claim 1, comprising a step of adding water to a reaction liquid obtained in the reaction step, then separating the obtained mixture into an organic layer and an aqueous layer, and conducting acid precipitation of the obtained aqueous layer to obtain a crude composition containing 4,4'-dihydroxybiphenyl-3,3'-dicarboxylic acid represented by the formula (2).

9. The method according to claim 8, comprising a step of further purifying the crude composition.

10. The method according to claim 9, wherein a purification step includes one or more steps selected from a group consisting of suspension washing, recrystallization, and reprecipitation.

11. The method according to claim 9, wherein the purification step includes a suspension washing step.

12. The method according to claim 9, wherein a solvent to be used in the purification step is one or more selected from a group consisting of water, methanol, ethanol, n-propyl alcohol, isopropyl alcohol, 2-ethylhexanol, ethylene glycol, glycerin, acetic acid, N-methyl-2-pyrrolidone, N,N-dimethylformamide, acetonitrile, acetone, xylene, toluene, methyl ethyl ketone, methyl isobutyl ketone, hexane, heptane, cyclohexanone, tetrahydrofuran, 4-methyltetrahydropyran, chloroform, dioxane, ethyl acetate, propyl acetate, and butyl acetate.

13. 4,4'-Dihydroxybiphenyl-3,3'-dicarboxylic acid represented by the formula (2), wherein a content of potassium is less than 100 ppm.
